# EUROPEAN PATENT SPECIFICATION

(11) **EP 2 943 246 B1**
(45) Date of publication and mention of the grant of the patent: **29.03.2017**
(21) Application number: 13704010.1
(22) Date of filing: 11.01.2013
(51) Int. Cl.: A61N 1/02, A61N 1/05

(54) **SYSTEM FOR NEURAL HEARING STIMULATION**
SYSTEM ZUR NEURALEN HÖRSTIMULATION
SYSTÈME POUR STIMULATION D'ÉCOUTE NEURALE

(43) Date of publication of application: 18.11.2015
(73) Proprietor: Advanced Bionics AG, 8712 Stäfa (CH)
(72) Inventor: FREDELAKE, Stefan, 30451 Hannover (DE); HAMACHER, Volkmar, 30657 Hannover (DE)
(74) Representative: Schwan Schorer & Partner mbB
(86) International application number: PCT/EP2013/050468
(87) International publication number: WO 2014/108201

(56) References cited:
- WO-A1-97/38653
- WO-A1-2012/161717
- WO-A2-2011/051831
- US-A1- 2010 161 000
- US-A1- 2011 064 241
- US-A1- 2011 077 698

## Description

The invention relates to a system for neural stimulation of a patient's hearing, such as by cochlea stimulation.

The sense of hearing in human beings involves the use of hair cells in the cochlea that convert or transduce acoustic signals into auditory nerve impulses. Hearing loss, which may be due to many different causes, is generally of two types: conductive and sensorineural. Conductive hearing loss occurs when the normal mechanical pathways for sound to excite the hair cells in the cochlea are impeded. These sound pathways may be impeded, for example, by damage to the auditory ossicles. Conductive hearing loss may often be overcome through the use of conventional hearing aids that amplify sound so that acoustic signals can reach the hair cells within the cochlea. Some types of conductive hearing loss may also be treated by surgical procedures.

Sensorineural hearing loss, on the other hand, is caused by the absence or destruction of the hair cells in the cochlea which are needed to transduce acoustic signals into auditory nerve impulses. People who suffer from severe to profound sensorineural hearing loss may be unable to derive significant benefit from conventional hearing aid systems, no matter how loud the acoustic stimulus is. This is because the mechanism for transducing sound energy into auditory nerve impulses has been damaged. Thus, in the absence of properly functioning hair cells, auditory nerve impulses cannot be generated directly from sounds.

To overcome sensorineural hearing loss, numerous auditory prosthesis systems (e.g., cochlear implant (CI) systems) have been developed. Auditory prosthesis systems bypass the hair cells in the cochlea by presenting electrical stimulation directly to the auditory nerve fibers. Direct stimulation of the auditory nerve fibers leads to the perception of sound in the brain and at least partial restoration of hearing function.

To facilitate direct stimulation of the auditory nerve fibers, a lead having an array of electrodes disposed thereon may be implanted in the cochlea of a patient. The electrodes form a number of stimulation channels through which electrical stimulation pulses may be applied directly to auditory nerves within the cochlea. An audio signal may then be presented to the patient by translating the audio signal into a number of electrical stimulation pulses and applying the stimulation pulses directly to the auditory nerve within the cochlea via one or more of the electrodes.

Typically, the audio signal, which usually is captured by a microphone, is divided into a plurality of analysis channels, each containing a frequency domain signal representative of a distinct frequency portion of the audio signal, wherein the frequency domain signal in each analysis channel may undergo signal processing, such as by applying channel-specific gain to the signals. The processed frequency domain signals are used for generating certain stimulation parameters according to which the stimulation signals in each stimulation channel is generated. The analysis channels are linked to the stimulation channels via channel mapping. The number of stimulation channels may correspond to the number of analysis channels, or there may be more stimulation channels than analysis channels, or there may be more analysis channels than stimulation channels. Various stimulation strategies are used, such as current steering stimulation (in order to stimulate a stimulation site located in between areas associated with two or more electrodes) and N-of-M stimulation (wherein stimulation current is only applied to N of M total stimulation channels during a particular stimulation frame).

An example for such a CI system with electrical cochlea stimulation is described in WO 2011/032021 A1.

Patients who are precluded from the use of a cochlear implant due to illness or injury damaging the cochlea or auditory nerve, may be provided with an auditory brainstem implant or an auditory midbrain implant. Such devices use similar technology as a cochlear implant, but instead of electrical stimulation being used to stimulate the cochlea, it is used to stimulate the brainstem or midbrain of the recipient.
CI users, especially non-experienced CI users, often complain that sounds are perceived as very loud when they switch-on their CI after a longer period of non-use, see for example J. Wolfe and E. Schafer, "Programming cochlea implants", Plural Publishing, Inc., 1st edition, 2010. As a consequence, CI users often decrease the volume by operating the manual volume control accordingly after the CI device has been turned on. Later, after a certain acclimatization period, the volume is increased by the CI users to the desired stable volume, wherein even the maximal position/setting of the volume control might be achieved. However, such manual volume increase might be annoying or difficult to the user, in particular for elderly CI users.

WO 2008/092183 A1 relates to a CI system, wherein patient attributes are collected and applied in sound data processing in a data processing resource external to the implanted part and body-worn part of the CI system. The external processing resource may be a mobile phone or TDA. It is mentioned that some patients are more sensitive to stimulation after periods of sleep with their prosthesis inactive, with such patients preferring a slow onset and amplitude limiting for loud sudden sounds during early morning that they may not wish at other times.

WO 97/38653 A1 relates to a cochlear implant system comprising a microphone, a sound processor, an implantable stimulation assembly and a control unit for controlling the sound processor. It is mentioned that the net current over the total period T of a bi-phased stimulation pulse should be kept at a minimum, and preferably equal to zero, so that, if the average value over a period T is found to differ from zero, then by means of a feedback element the time duration of the first and/or second phase will be automatically adjusted or the currents in the positive period will be adapted with respect to the currents in the negative period such that the net current is zero.

In addition, loudness perception of some patients also may vary on a scale significantly longer than just a few minutes or hours. In particular, when a patient uses his CI device for the first time at all or after a fitting session wherein the device settings have been significantly changed, some patients prefer relatively low stimulation levels while later, due to acclimatization effects, these low levels may be no longer sufficient for an appropriate perception of sounds, which requires the CI user to manually increase the volume. However, if the maximal possible volume is achieved and the stimulation levels became too low for optimal perception again, the CI user needs to obtain a new fitting of the device, i.e. to consult the audiologist; such action often is time-consuming both for the patient who may need to travel for several hours to the audiologist's place and the audiologist who has to work with an additional CI patient.

Similar issues exist with regard to other auditory prosthesis for neural stimulation, such as auditory brain stem implants and auditory midbrain implants.

A device according to the preamble of claim 1 is known from US2010/161000.

It is an object of the invention to provide for an auditory prosthesis device for neural hearing stimulation which is particularly easy to use.

According to the invention, this object is achieved by a device as defined in claim 1.

The invention is beneficial in that, by recording the time history of the operation of a manual volume control provided for adding a manually variable level to a base level of the neural stimulation signal and by controlling at least one parameter level used in the generation of the neural stimulation signal from the input audio signal such that, during an adjustment period, the value of the at least one parameter level is automatically changed according to the recorded volume control operation history, the auditory prosthesis device can be automatically adjusted to the patient's preferences, so that the need for a new fitting session could be at least reduced.

Further preferred embodiments of the invention are defined in the dependent claims. Hereinafter, examples of the invention will be illustrated by reference to the attached drawings, wherein:
- Fig. 1: is a schematic view of an example of a CI system according to the invention;
- Fig. 2: is a schematic cross-sectional view of a human cochlea with marked stimulation sites;
- Fig. 3: is a block diagram of the signal processing structure of a CI system according to the invention;
- Fig. 4: is a diagram, wherein an example of an automatic increase of the MCL value during an adjustment period after turning-on of a CI-system is shown, which example does not form part of the invention, with the time constant of the increase depending on the duration of the preceding turning-off period of the CI-system;
- Fig. 5: is a diagram like Fig. 4, wherein an alternative example is shown, wherein the initial MCL value depends on the duration of the preceding turning-off period of the CI-system, which example does not form part of the invention;
- Fig. 6: is a diagram, wherein an example an automatic increase of the MCL value during an adjustment period after a fitting event of a CI-system is shown, which example does not form part of the invention,; and
- Fig. 7: is a diagram, wherein an example of the MCL value as a function of time during an adjustment period after a fitting event of a CI-system is shown, wherein the MCL is adjustable by a manual volume control and wherein the maximum MCL is automatically adjusted according to the operation history of the manual volume control.

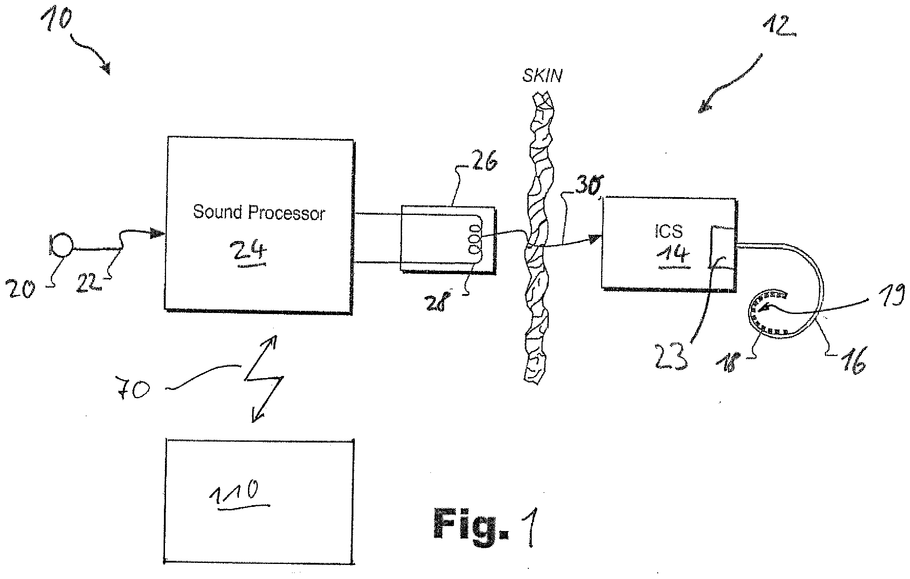

In Fig. 1 an example of a cochlear implant system is shown schematically. The system comprises a sound processing sub-system 10 and a stimulation sub-system 12. The sound processing sub-system 10 serves to detect or sense an audio signal and divide the audio signal into a plurality of analysis channels each containing a frequency domain signal (or simply "signal") representative of a distinct frequency portion of the audio signal. A signal level value and a noise level value are determined for each analysis channel by analyzing the respective frequency domain signal, and a noise reduction gain parameter is determined for each analysis channel as a function of the signal level value and the noise level value of the respective analysis channel. Noise reduction is applied to the frequency domain signal according to the noise reduction gain parameters to generate a noise reduced frequency domain signal. Stimulation parameters are generated based on the noise reduced frequency domain signal and are transmitted to the stimulation sub-system 12.

Stimulation sub-system 12 serves to generate and apply electrical stimulation (also referred to herein as "stimulation current" and/or "stimulation pulses") to stimulation sites at the auditory nerve within the cochlear of a patient in accordance with the stimulation parameters received from the sound processing sub-system 10. Electrical stimulation is provided to the patient via a CI stimulation assembly 18 comprising a plurality of stimulation channels, wherein various known stimulation strategies, such as current steering stimulation or N-of-M stimulation, may be utilized.

As used herein, a "current steering stimulation strategy" is one in which weighted stimulation current is applied concurrently to two or more electrodes by an implantable cochlear stimulator in order to stimulate a stimulation site located in between areas associated with the two or more electrodes and thereby create a perception of a frequency in between the frequencies associated with the two or more electrodes, compensate for one or more disabled electrodes, and/or generate a target pitch that is outside a range of pitches associated with an array of electrodes.

As used herein, an "N-of-M stimulation strategy" is one in which stimulation current is only applied to N of M total stimulation channels during a particular stimulation frame, where N is less than M. An N-of-M stimulation strategy may be used to prevent irrelevant information contained within an audio signal from being presented to a CI user, achieve higher stimulation rates, minimize electrode interaction, and/or for any other reason as may serve a particular application.

The stimulation parameters may control various parameters of the electrical stimulation applied to a stimulation site including, but not limited to, frequency, pulse width, amplitude, waveform (e.g., square or sinusoidal), electrode polarity (i.e., anode-cathode assignment), location (i.e., which electrode pair or electrode group receives the stimulation current), burst pattern (e.g., burst on time and burst off time), duty cycle or burst repeat interval, spectral tilt, ramp on time, and ramp off time of the stimulation current that is applied to the stimulation site.

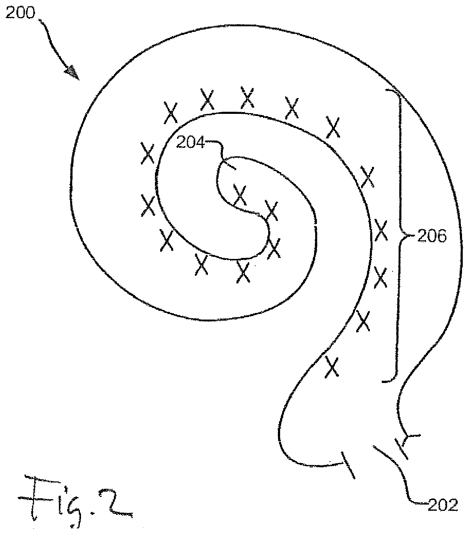

Fig. 2 illustrates a schematic structure of the human cochlea 200. As shown in Fig. 2, the cochlea 200 is in the shape of a spiral beginning at a base 202 and ending at an apex 204. Within the cochlea 200 resides auditory nerve tissue 206, which is denoted by Xs in Fig. 2. The auditory nerve tissue 206 is organized within the cochlea 200 in a tonotopic manner. Low frequencies are encoded at the apex 204 of the cochlea 200 while high frequencies are encoded at the base 202. Hence, each location along the length of the cochlea 200 corresponds to a different perceived frequency. Stimulation subsystem 12 is configured to apply stimulation to different locations within the cochlea 200 (e.g., different locations along the auditory nerve tissue 206) to provide a sensation of hearing.

Returning to Fig. 1, sound processing subsystem 10 and stimulation subsystem 12 may be configured to operate in accordance with one or more control parameters. These control parameters may be configured to specify one or more stimulation parameters, operating parameters, and/or any other parameter as may serve a particular application. Exemplary control parameters include, but are not limited to, most comfortable current levels ("M levels"), threshold current levels ("T levels"), dynamic range parameters, channel acoustic gain parameters, front and backend dynamic range parameters, current steering parameters, amplitude values, pulse rate values, pulse width values, polarity values, filter characteristics, and/or any other control parameter as may serve a particular application.

In the example shown in Fig. 1, the stimulation sub-system 12 comprises an implantable cochlear stimulator ("ICS") 14, a lead 16 and the stimulation assembly 18 disposed on the lead 16. The stimulation assembly 18 comprises a plurality of "stimulation contacts" 19 for electrical stimulation of the auditory nerve. The lead 16 may be inserted within a duct of the cochlea in such a manner that the stimulation contacts 19 are in communication with one or more stimulation sites within the cochlea, i.e. the stimulation contacts 19 are adjacent to, in the general vicinity of, in close proximity to, directly next to, or directly on the respective stimulation site.

In the example shown in Fig. 1, the sound processing sub-system 10 is designed as being located external to the patient; however, in alternative examples, at least one of the components of the sub-system 10 may be implantable.

In the example shown in Fig. 1, the sound processing sub-system 10 comprises a microphone 20 which captures audio signals from ambient sound, a microphone link 22, a sound processor 24 which receives audio signals from the microphone 20 via the link 22, and a headpiece 26 having a coil 28 disposed therein. The sound processor 24 is configured to process the captured audio signals in accordance with a selected sound processing strategy to generate appropriate stimulation parameters for controlling the ICS 14 and may include, or be implemented within, a behind-the-ear (BTE) unit or a portable speech processor ("PSP"). In the example of Fig. 1 the sound processor 24 is configured to transcutaneously transmit data (in particular data representative of one or more stimulation parameters) to the ICS 14 via a wireless transcutaneous communication link 30. The headpiece 26 may be affixed to the patient's head and positioned such that the coil 28 is communicatively coupled to the corresponding coil (not shown) included within the ICS 14 in order to establish the link 30. The link 30 may include a bidirectional communication link and/or one or more dedicated unidirectional communication links. According to an alternative embodiment, the sound processor 24 and the ICS 14 may be directly connected by wires.

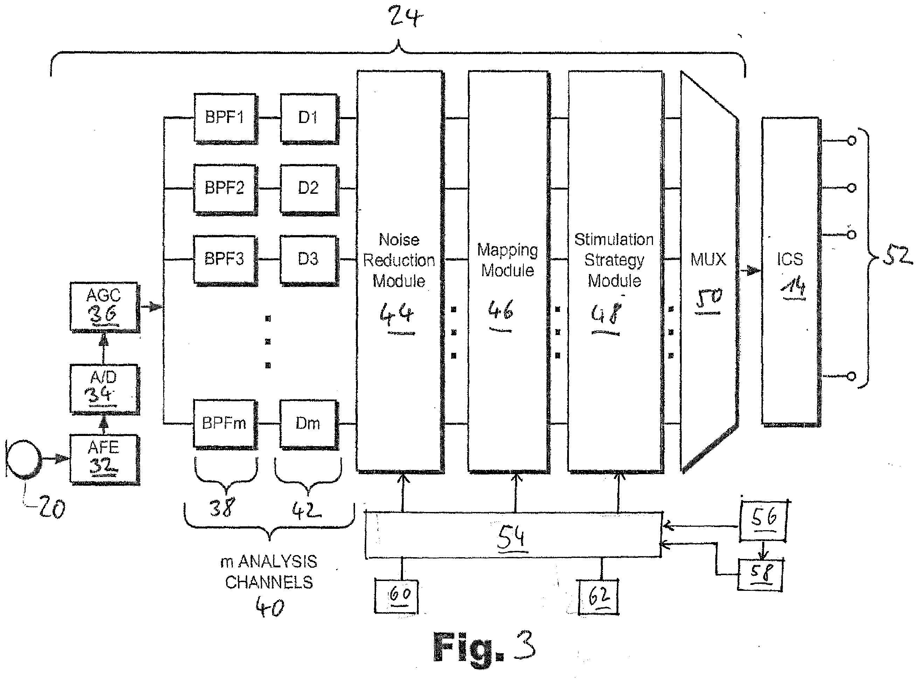

In Fig. 3 a schematic example of a sound processor 24 is shown. The audio signals captured by the microphone 20 are amplified in an audio front end circuitry 32, with the amplified audio signal being converted to a digital signal by an analog-to-digital converter 34. The resulting digital signal is then subjected to automatic gain control using a suitable automatic gain control (AGC) unit 36.

After appropriate automatic gain control, the digital signal is subjected to a plurality of filters 38 (for example, band-pass filters) which are configured to divide the digital signal into *m* analysis channels 40, each containing a signal representative of a distinct frequency portion of the audio signal sensed by the microphone 20. For example, such frequency filtering may be implemented by applying a Discrete Fourier Transform to the audio signal and then divide the resulting frequency bins into the analysis channels 40.

The signals within each analysis channel 40 are input into an envelope detector 42 in order to determine the amount of energy contained within each of the signals within the analysis channels 40 and to estimate the noise within each channel. After envelope detection the signals within the analysis channels 40 are input into a noise reduction module 44, wherein the signals are treated in a manner so as to reduce noise in the signal in order to enhance, for example, the intelligibility of speech by the patient. Examples of the noise reduction module 44 are described e.g. in WO 2011/032021 A1.

The noise reduced signals are supplied to a mapping module 46 which serves to map the signals in the analysis channels 40 to the stimulation channels. For example, signal levels of the noise reduced signals may be mapped to amplitude values used to define the electrical stimulation pulses that are applied to the patient by the ICS 14 via M stimulation channels 52. For example, each of the *m* stimulation channels 52 may be associated to one of the stimulation contacts 19 or to a group of the stimulation contacts 19.

The sound processor 24 further comprises a stimulation strategy module 48 which serves to generate one or more stimulation parameters based on the noise reduced signals and in accordance with a certain stimulation strategy (which may be selected from a plurality of stimulation strategies). For example, stimulation strategy module 48 may generate stimulation parameters which direct the ICS 14 to generate and concurrently apply weighted stimulation current via a plurality of the stimulation channels 52 in order to effectuate a current steering stimulation strategy. Additionally or alternatively the stimulation strategy module 48 may be configured to generate stimulation parameters which direct the ICS 14 to apply electrical stimulation via only a subset N of the stimulation channels 52 in order to effectuate an N-of-M stimulation strategy.

The sound processor 24 also comprises a multiplexer 50 which serves to serialize the stimulation parameters generated by the stimulation strategy module 48 so that they can be transmitted to the ICS 14 via the communication link 30, i.e. via the coil 28.

The sound processor 24 operates in accordance with at least one control parameter which is set by a control unit 54. Such control parameters may be the most comfortable listening current levels (MCL), also referred to as "M levels", threshold current levels (also referred to as "T levels"), dynamic range parameters, channel acoustic gain parameters, front and back end dynamic range parameters, current steering parameters, amplitude values, pulse rate values, pulse width values, polarity values and/or filter characteristics. Examples of such auditory prosthesis devices, as described so far, can be found, for example, in WO 2011/032021 A1.

According to examples which do not form part of the present invention, the control unit of the sound processor is adapted to control at least one of the parameters used in the generation of the neural stimulation signal in the sound processor such that, during an adjustment period, the value of the parameter is automatically changed from a first value to a second value as a function of the time having passed since a reference point in time, i.e. since a certain event. Time need not be directly monitored, but may be monitored indirectly by monitoring time related parameters, such as the power or voltage supplied by the battery (which decreases as a function of time) or the number of pulses that have been presented (which increases as a function of time). Typically, the parameter value is automatically changed during the adjustment period from the first value to the second value as a monotonous function of the time having passed since the reference point in time. Typically, after the adjustment period the value of the parameter remains at the second value. Preferably, the parameter is a level parameter of the neural stimulation signal, such as the MCL or M-level. More generally, the level parameter may be a reference level which is used by the sound processor to determine the output level of the neural stimulation signal as a function of the level and the spectrum of the input audio signal.

Preferably, the increase of the parameter value during the adjustment period is asymptotic.

According to one example, the adjustment period begins when the device is turned on, such as by operating a switch 56, after a turn-off period of the device, i.e. the reference point in time or event is the time of the turning-on of the device. Preferably, the device comprises means for determining the duration of the turn-off period, such as a timer or clock 58, with the function of time of the parameter adjustment during the adjustment period being selected as a function of the determined duration of the turn-off period. The timer/clock 58 may be implemented in the sound processor 24 and may be supplied by the battery of the sound processing sub-system 10.

According to one example, the second value of the parameter to be adjusted may be independent of the determined duration of the turn-off period, whereas the first value of the parameter to be adjusted may be selected as a function of the determined duration of the turn-off period; for example, the first value may decrease with increasing determined duration of the turn-off period. Alternatively or in addition, the time constant of the automatic increase of the parameter value during the adjustment period may depend on the determined duration of the turn-off period; for example, the time constant of the automatic increase of the parameter value during the adjustment period may increase with increasing determined duration of the turn-off period.

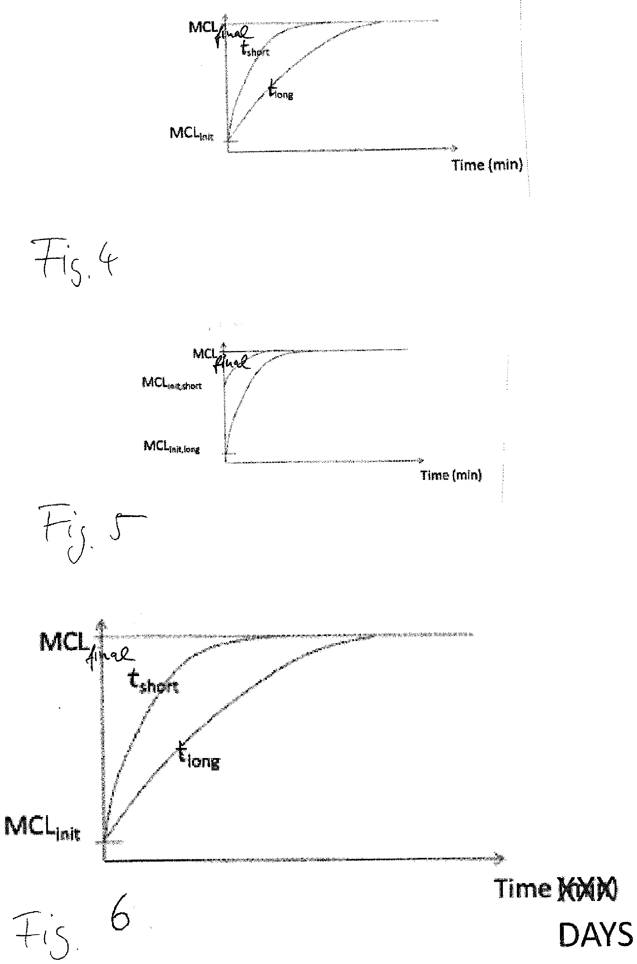

Examples of such automatic adjustment of the volume setting of an auditory prosthesis device are illustrated in Figs. 4 and 5, wherein the set value of the MCL is shown as a function of time after turning on of the device after a certain turn-off period.

In the example of Fig. 4, the MCL value is monotonously increased according to an asymptotic function having a time constant *t* from an initial value MCLᵢₙᵢₜ (first value) to a final value MCL_{final} (second value). In the example of Fig. 4 the initial value MCLᵢₙᵢₜ is selected independently of the duration of the preceding turn-off period, whereas the time constant *t* of the increase depends on the duration of the turn-off period: after a relatively long turn-off period (for example, 8 hours) a relatively long time constant *t*_{long} is selected, resulting in a relatively slow increase of the MCL value, whereas for a relatively short turn-off period (for example, only one or two hours) a relatively short time constant *t*ₛₕₒᵣₜ is selected, resulting in a relatively fast increase of the MCL value. An example of a use situation with a relatively long turn-off period is the sleeping of the patient during night-time, and an example of a use situation with a relatively short turn-off period when the patient has been swimming.

In the examples of Fig. 5, a relatively low initial value MCL_{init, long} is selected in case of a relatively long turn-off period, whereas a relatively high initial value MCL_{init, short} is selected in case of a relatively short turn-off period, with the time constant of the increase not depending on the duration of the preceding turn-off period.

However, the examples of Fig. 4 and 5 also may be combined by selecting both the initial MCL value and the time constant as a function of the duration of the preceding turn-off period. Examples of other parameters which may be automatically changed during the adjustment period as a function of time, are the gain, noise reduction parameters or the sensitivity of the microphones.

The examples of Fig. 4 and 5 relate to a "short-term" adaptation of the parameter setting in that the duration of the automatic adjustment, i.e. the duration of the adjustment period, is within the range of minutes, such as 10 minutes. For example, if the device was not used for eight hours during night, the volume setting may be initially, i.e. at the time when the device is turned on, decreased by about 50% to "MCLᵢₙᵢₜ" and then may asymptotically reach the standard volume setting "MCL_{final}" after about 10 minutes.

The time constant *t* of the automatic MCL increase also may be individually adjusted according to the user preferences. For example, for a user who usually increases the manual volume control during the course of a day a relatively long time constant may be selected in order to mimic this user preference.

Further, in order to take into account a long term acclimatization of the user to new parameter settings after a fitting session, the time constant may be automatically adjusted as a function of the time having passed since the last fitting session of the device. For example, the time constant of the increase may be gradually shortened after use of the device for several weeks or months after the last fitting session. In this regard, the timer 58 may be used not only for determining the duration of the last turn-off period but also for determining the time having passed since the last fitting session.

In order to avoid very loud initial perception upon turning on of the device, the initial MCL value may be selected as a function of the input sound level prevailing at the time when the device is turned on; to this end, the initial parameter value decreases with increasing level of the input audio signal at the time when the device is turned on, i.e. the higher the input audio signal level is upon turning-on of the device, the lower the selected initial MCL value will be. Alternatively, other parameters which are directly related to loudness perception, such as sensitivy parameters, mapping parameters, noise reduction parameters, and temporal enhancer parameters may be treated similarly, i.e. they may be selected as a function of the input sound level prevailing at the time when the device is turned on in such a manner that the loudness perception decreases with increasing level of the input audio signal at the time when the device is turned on.

According to one embodiment, the device may be provided with a user interface 60 for manual override of settings of the automatic increase of the parameter value during the adjustment period, so that the user, for example, may decide that a more rapid increase of the MCL value to the standard value is desired than provided by the automatic short term adjustment provided by the control unit 54.

In addition, as already mentioned above, the device typically is provided with a manual volume control 62 which adds a manually adjustable level to the MCL value provided by the automatic parameter control.

Alternatively or in addition to the short term adaptation of signal processing parameters a long-term adaptation of such parameters may be implemented in the control unit. Typically, the time constants of such long-term adaptation, i.e. the duration of the adjustment period in case of long term adaptation, may be days, weeks or months.

According to a first example, the long term adaptation may be similar to the short term adaptation in that the parameter value is adjusted during an adjustment period as a function of the time having passed since a reference point in time; however, while for short term adaptation the reference point in time is the time the device has been turned on, for the long term adjustment the reference point in time is the time of the last fitting of the device.

An example is shown in Fig. 6 for two different time constants *t*ₛₕₒᵣₜ and *t*_{long}; the time constant may be individually selected according to the preferences of the user. Such long term adjustment is useful for taking into account a gradual change of the loudness perception of the user after first time (or most recent) fitting of the device in the course of days, weeks or months after the fitting event. The target value of the parameter (i.e. the target MCL value in Fig. 6) may be either set by the audiologist manually or it may be automatically set by the fitting software; the target value may be estimated from physiologic measures or experiences. The target value should be carefully set according to defined rules in order to avoid uncomfortable loudness or pain. In case that unexpectedly uncomfortable loudness or pain occurs during the adjustment period, the user may override the automatic control by operation of the user interface 60 in order to reduce, for example, the MCL value or to stop its automatic increase.

As already mentioned above with regard to the short term adjustment, the daily short term adjustment and the long term adjustment may be combined.

According to a type of long term adjustment which forms part of the present invention, the control unit of the sound processor is adapted to record a time history of the operation of the manual volume control 62 and to automatically adjust a level parameter value of the sound processor. In this case, in contrast to the examples described so far, the automatic parameter adjustment during the adjustment period is not a function of the time having passed since a reference point in time, but rather the automatic adjustment of the parameter value occurs according to the observed/recorded history of the manual operation of the device by the user, in particular according to the manual volume control operation history.

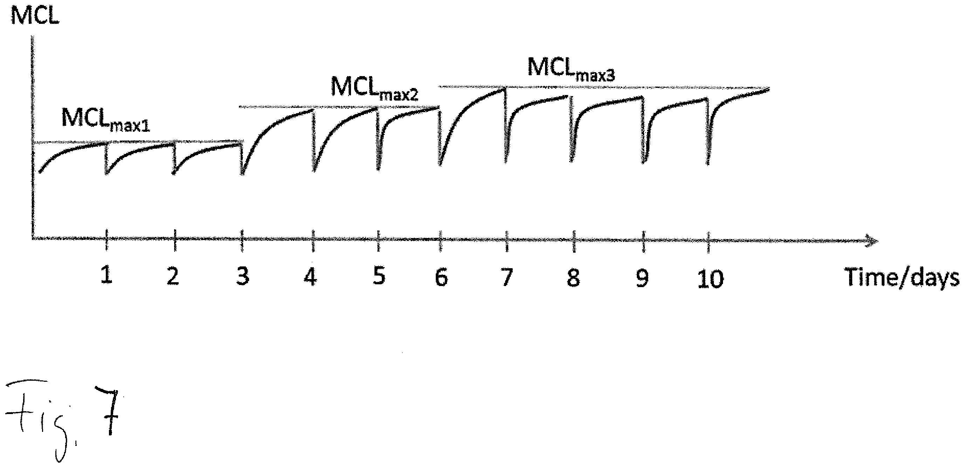

An example is shown in Fig. 7, wherein the effective MCL value (i.e. including the MCL adjustment via the manual volume control) is shown as a function of time for several days.

According to this embodiment the position/setting of the manual volume control is monitored during a certain time window in order to obtain a history of the operation of the volume control and then level parameter values, such as the MCL value, may be adjusted accordingly in an automatic manner. For example, if it is found that during a first observation time window the volume control was operated several times for achieving a maximal volume (i.e. the user has attempted several times to achieve the maximal MCL), the MCL value set by the control unit may be increased by a certain amount, for example by 10 %. If it is found then that during the next observation time window the volume control still was operated several times in a manner to achieve the maximal level, the MCL value set by the control unit may be increased again, but usually this time by a smaller amount, for example by 5 %. This added amount may be exponentially decreased within a defined time period in order to avoid over-loud stimulation.

In the example of Fig. 7, the time window of the observation/monitoring period is three days, and it is found that during the first three days on every day, after some time, the volume control has been turned to its maximum position. Consequently, the MCL value is increased by the control unit on the fourth day by a certain amount. However, the user still adjusts the volume control to its maximal position after some time of the day during days four to six. As a consequence, on the seventh day the MCL value set by the control unit is again increased, but this time by a lower amount. Now the MCL value has reached a level on which the user feels no further desire to adjust the volume control to its maximal position. Consequently, the control unit now does not further increase the MCL value.

In both long term adjustment and short term adjustment the parameter to be adjusted may not only be a level parameter, such as the MCL, but alternatively or in addition audio processing parameters like the stimulation pulse width, the stimulation pulse rate, the inter-pulse interval, the number of electrodes used for stimulation, the sensitivity of a microphone arrangement used for providing the input signal, the linear input gain, the AGC (automatic gain control) settings and noise reduction settings may be automatically adjusted.

For short term adjustment not only "M-levels" but also other parameters which affect loudness could be changed in addition or alternatively, such as the microphone sensitivity or the AGC settings. For example, after some hours of non-use of the device, the sensitivity of the microphones may be decreased or the AGC attack time constants may be set to relatively low values in order to reduce impulsive noises. After turning on the device, the sensitivity of the microphone and/or the AGC attack time constant may be increased during the adjustment period as a function of the time having passed since the device has been turned on.

Further, both during short term or long term adjustment, telemetry data obtained from electric measurements at neural stimulation sites conducted via the stimulation assembly and transmitted from the ICS via a transcutaneous link to the external part 10, may be used by the control unit in order to take into account such data when adjusting the parameter values, in particular when adjusting level parameter values.

Typically, the auditory prosthesis device will be a cochlear implant device, with the neural stimulation signal being an auditory nerve stimulation signal. However, the present invention also may be applied to other neural stimulation devices, such as auditory brainstem implants or auditory mid brain implants.

The device may be part of a binaural, bilateral and/or bimodal system comprising auditory prosthesis devices at both ears. For example, one of the devices may be an electric stimulation device, whereas the other is a hearing aid, i.e. uses acoustic stimulation. In this case, the loudness perception on one of the devices may depend on the use of the device at the other ear. Consequently, the control unit of the auditory prosthesis device at one of the ears may take into account not only the time having passed since a certain event, such as turning on, of that device, but in addition also the time having passed since a certain event happened to the device worn at the other ear, such as the time having passed since the time when the device at the other ear has been turned on. In general, in the parameter adjustment in the device at one of the ears information from both devices may be used and synchronized in order to achieve optimal adjustment of the devices. Such synchronization, of course, requires the implementation of a respective link for data exchange between the devices.

In the example of Fig. 1 an auditory prosthesis device worn at the other (i.e. contralateral) ear is indicated at 110, and a communication link between the device 110 and the CI device 10, 12 is indicated at 70.

## Claims

1. An auditory prosthesis device for neural stimulation of a patient's hearing, comprising
means (20) for providing an input audio signal;
a sound processor (24) for generating a neural stimulation signal from the input audio signal,
an implantable stimulation assembly (12) for stimulation of the patient's hearing according to the neural stimulation signal,
a control unit (54) for controlling the sound processor, the control unit comprising a manual volume control (62) for adding a manually variable level to a level value set by the control unit, **characterized in that** said control unit further comprises means for recording a history of the operation of the volume control during a monitoring period,
wherein the control unit is adapted to control at least one level parameter used in the generation of the neural stimulation signal from the input audio signal such that, during an adjustment period, the value of the at least one level parameter is automatically changed according to the recorded volume control operation history.

2. The device of claim 1, wherein the parameter is the MCL.

3. The device of one of claims 1 and 2, wherein the value of the level parameter is increased by a certain increment when the volume control has reached the maximal level setting at least once during the monitoring period.

4. The device of claim 3, wherein the value of the level parameter is kept constant when the volume control has not reached the maximal level setting during the monitoring period.

5. The device of one of claims 3 and 4, wherein the increment is reduced as a function of time.

## Patentansprüche

1. Hörprothesengerät zur neuralen Stimulation des Gehörs eines Patienten, mit
Mitteln (20) zum Bereitstellen eines Eingangsaudiosignals;
einem Schallprozessor (24) zum Erzeugen eines neuralen Stimulationssignals aus dem Eingangsaudiosignal;
einer implantierbaren Stimulationsbaugruppe (12) zum Stimulieren des Gehörs des Patienten gemäß dem neuralen Stimulationssignal;
einer Steuereinheit (54) zum Steuern des Schallprozessors, die eine manuelle Volumensteuerung (62) zum Hinzufügen eines manuell variablen Pegels zu einem von der Steuereinheit festgelegten Pegelwert aufweist,
**dadurch gekennzeichnet, dass**
die Steuereinheit ferner Mittel zum Aufzeichnen einer Betriebshistorie der Lautstärkesteuerung während einer Überwachungsperiode aufweist,
wobei die Steuereinheit ausgebildet ist, um mindestens einen Pegelparameter, der zur Erzeugung des neuralen Stimulationssignals aus dem Eingangsaudiosignal verwendet wird, so zu steuern, dass während einer Anpassungsperiode der Wert des mindestens einen Pegelparameters gemäß der aufgezeichneten Betriebshistorie der Lautstärkesteuerung automatisch verändert wird.

2. Gerät gemäß Anspruch 1, wobei es sich bei dem Parameter um den MCL handelt.

3. Gerät gemäß einem der Ansprüche 1 oder 2, wobei der Wert des Pegelparameters um einen bestimmten Zuwachs erhöht wird, wenn die Lautstärkesteuerung die maximale Pegeleinstellung während der Überwachungsperiode mindestens einmal erreicht hat.

4. Gerät gemäß Anspruch 3, wobei der Wert des Pegelparameters konstant gehalten wird, wenn die Lautstärkesteuerung die maximale Pegeleinstellung während der Überwachungsperiode nicht erreicht hat.

5. Gerät gemäß einem der Ansprüche 3 oder 4, wobei der Zuwachs als Funktion der Zeit verringert wird.

## Revendications

1. Dispositif de prothèse auditive pour une stimulation neurale de l'écoute d'un patient, comprenant
un moyen (20) pour fournir un signal audio d'entrée ;
un processeur de son (24) pour générer un signal de stimulation neurale à partir du signal audio d'entrée,
un ensemble de stimulation implantable (12) permettant une stimulation de l'écoute d'un patient en fonction du signal de stimulation neurale,
une unité de commande (54) pour commander le processeur de son, l'unité de commande comprenant un réglage de volume manuel (62) pour ajouter un niveau variable manuellement à une valeur de niveau définie par l'unité de commande,
**caractérisé en ce que**
ladite unité de commande comprend en outre un moyen pour enregistrer un historique de l'opération de réglage de volume pendant une période de surveillance,
dans lequel l'unité de commande est conçue pour commander au moins un paramètre de niveau utilisé lors de la génération du signal de stimulation neurale à partir du signal audio d'entrée de telle sorte que, pendant une période de réglage, la valeur du ou des paramètres de niveau soit changée automatiquement en fonction de l'historique de l'opération de réglage de volume enregistré.

2. Dispositif selon la revendication 1, dans lequel le paramètre est le MCL.

3. Dispositif selon l'une quelconque des revendications 1 et 2, dans lequel la valeur du paramètre de niveau est accrue par un certain incrément lorsque le réglage de volume a atteint le réglage de niveau maximal au moins une fois pendant la période de surveillance.

4. Dispositif selon la revendication 3, dans lequel la valeur du paramètre de niveau est gardée constante lorsque le réglage de volume n'a pas atteint le réglage de niveau maximal au moins pendant la période de surveillance.

5. Dispositif selon l'une quelconque des revendications 3 et 4, dans lequel l'incrément est réduit en fonction du temps.
